# EUROPEAN PATENT SPECIFICATION

(11) **EP 1 977 242 B1**
(45) Date of publication and mention of the grant of the patent: **03.08.2016**
(21) Application number: 07762849.3
(22) Date of filing: 26.01.2007
(51) Int. Cl.: G01N 33/53

(54) **LATERAL FLOW IMMUNOASSAY WITH ENCAPSULATED DETECTION MODALITY**
QUERFLUSS-IMMUNTEST MIT EINGEKAPSELTER ERKENNUNGSMODALITÄT
IMMUNOESSAI DE FLUX LATÉRAL FAISANT INTERVENIR DES PARTICULES ENCAPSULÉES EN TANT QUE MODALITE DE DÉTECTION

(30) Priority: 27.01.2006 US 763034 P
(43) Date of publication of application: 08.10.2008
(73) Proprietor: Becton Dickinson and Company, Franklin Lakes, NJ 07417-1880 (US)
(72) Inventor: NATAN, Michael, Los Altos, California 94024 (US); CROMER, Remy, San Jose, California 95130 (US); DOERING, William, Mountain View, California 94043 (US); CORISH, Peter, Oxfordshire Oxfordshire OX5 1PF (GB)
(74) Representative: Eder, Michael
(86) International application number: PCT/US2007/061136
(87) International publication number: WO 2007/090058

(56) References cited:
- WO-A-2006/073439
- US-A1- 2005 112 703
- US-A1- 2005 130 240
- US-A1- 2005 158 877
- US-A1- 2007 087 383
- US-B2- 6 884 628
- NI J ET AL: "Immunoassay readout method using extrinsic Raman labels adsorbed on immunogold colloids." ANALYTICAL CHEMISTRY 1 NOV 1999, vol. 71, no. 21, 1 November 1999 (1999-11-01), pages 4903-4908, XP002511910 ISSN: 0003-2700

## Description

### TECHNICAL FIELD

The present invention relates to a system and method for performing a lateral flow immunoassay (LFI), in particular a LFI employing encapsulated particles which use SERS tags as the detection modality.

### BACKGROUND OF THE INVENTION

A lateral flow immunoassay (LFI) is a popular platform for rapid and simple qualitative diagnostic testing. LFI's were introduced in the late 1980's. A typical LFI includes a thin substrate or membrane manufactured from cellulose nitrate or a similar material. Typically, the application of a sample to a conjugation pad on one portion of the substrate rehydrates dried reagents and initiates immunoreactions needed for the detection of an antigen of interest. A representative basic LFI is a pregnancy test of which several hundred thousand individual test strips are produced each year. After the sample is applied to the LFI conjugation pad, the sample and detection particles bound to the antigen wick up the test strip substrate through capillary action to a test marker portion of the substrate. If the antigen of interest is present, antibodies placed at the test marker capture the previously bound detection particles. In the case of a positive test, the bound detection particles become visible at the test marker. The visible test marker is typically a colored line, dot or symbol.

Typical LFI devices are quite simple to use in a variety of environments, require minimal to no sample preparation and produce a rapid result. However, a typical LFI is a relatively imprecise and purely qualitative test. High sensitivity, qualitative analysis or quantitative results are difficult or impossible to obtain with typical prior art LFI devices.

Traditionally, many LFI devices have utilized Au nanoparticles with diameters ranging from 20-40 nanometers as the detection modality. Appropriate antibodies are adsorbed directly to the Au surface before the particles are placed and bonded to the conjugation pad of the LFI. Positive tests may be indicated by the capture of these Au nanoparticles on the test line. Then visual detection may be used to observe the highly light scattering nature of the nanoparticles bound to the test line. Unfortunately, visual detection of the particles bound to the test line can be obscured by blood, soil or other contaminants.

The use of larger Au nanoparticles would increase the sensitivity of LFI tests because the scattering and absorbance of Au nanoparticles both increase in a non-linear fashion with increasing size. Since the steric hindrance of the nanoparticles themselves limits the ability of passively adsorbed antibodies to effectively bind antigen, there is a sensitivity tradeoff associated with merely increasing particle size. The best prior art combination of particle visibility and antibody activity usually comes with Au particles having a diameter of about 40 nm. Other metals, such as Ag, might increase sensitivity as well as a function of the higher extinction of Ag. Ag nanoparticles typically do not serve as well as Au however, to passively adsorb antibodies.

A crucial property of the detection particles in an LFI is their ability to completely release from a dried state on the conjugate pad. Ideally, this release is somewhat slow, thus allowing antigen and bound particles to subsequently bind to the capture line before the particles diffuse past the line. Prior art Au nanoparticles provide no choices of surface chemistry designed to optimize this release. Furthermore, the chemistry of prior art Au nanoparticles can not be tailored to minimize the non-specific adsorption of the particles to the substrate in which the assay is performed. Current practice is somewhat limited to the addition of blockers and additives to the buffers and substrate used in the assay.

Typical prior art LFI devices are limited by design to a single capture antibody. Thus, these devices are limited in use to the detection of a single species. There are examples of LFI tests with limited multiplexing capabilities. Typically the multiple antibodies selected to perform distinct tests are each associated with a separate capture line. Thus, the prior art teaches spatial multiplexing. In use, visual observation of the spatially separate capture lines allows, in a limited fashion, for a simple multiplexed qualitative test. Such a spatially multiplexed format, however, requires that the detection modality associated with the capture line positioned furthest from the conjugate pad diffuse through the other capture lines before a test can be read. Thus, spatial multiplexing can be problematic because of interference issues resulting in decreased sensitivity.

The present invention is directed toward overcoming one or more of the problems discussed above.

### SUMMARY OF THE INVENTION

The present invention provides a lateral flow immunoassay featuring encapsulated metal particles. The encapsulated particles use SERS nanotags as the detection modality. The use of encapsulated SERS tags as a detection modality, increases the sensitivity of an LFI prepared for visual reading and introduces the ability to obtain substantially more sensitive qualitative results or quantitative results through the analysis of a SERS spectrum read from an LFI prepared in accordance with the present invention. The use of SERS as detection modality also enhances the ability of an LFI device to be used for a multiplexed test. Other aspects of the present invention include LFI devices specifically configured to test whole blood, a reader for the detection and interpretation of a multiplexed assay and the hardware and software components used to implement the reader.

### BRIEF DESCRIPTION OF THE DRAWINGS

Fig. 1 shows an LFI consistent with the present invention demonstrating visual detection efficiency when compared to a commercially available prior art LFI at various sample dilutions;
Fig. 2 graphically shows the relative SERS signal collected at the test line of an LFI device consistent with the present invention for various sample dilutions;
Fig. 3 graphically shows the SERS signal obtained from an LFI kit as a function of the dilution of a positive control for RSV;
Fig. 4 graphically shows the SERS signal obtained from an LFI kit as a function of the dilution of a positive control for Flu A;
Fig. 5 graphically shows the SERS signal obtained from an LFI kit as a function of the dilution of a positive control for Flu B;
Fig. 6 graphically shows the SERS signal of multiplexed conjugates as a function of RSV antigen concentration;
Fig. 7 graphically shows the SERS signal of multiplexed conjugates as a function of Influenza A antigen concentration; and
Fig. 8 graphically shows the SERS signal of multiplexed conjugates as a function of Influenza B antigen concentration.
Fig. 9 is a computer screen capture of spectra obtained during multiplexed assay calibration.
Fig. 10 is a computer screen capture showing representative spectra data.
Fig. 11 is a computer screen capture showing a representative calibration curve for Flu B.
Fig. 12 is a computer screen capture showing a representative calibration curve for Flu A.
Fig. 13 is a computer screen capture showing a representative calibration curve for RSV.
Fig. 14 is a graph of assay performance with various tags.
Fig. 15 is a schematic scan diagram.
Fig. 16 is a graph of an assay featuring internal calibration.

### DETAILED DESCRIPTION OF THE INVENTION

The present invention provides a lateral flow immunoassay device, testing methods and associated equipment. The present LFI features the use of encapsulated particles as the detection modality instead of the metal nanoparticles of prior LFI devices. In one embodiment of the present invention, the encapsulated detection particles are SERS tags as described in U.S. Patent No. 6,514,767, entitled "surface Enhanced Spectroscopy-Active Composite Nanoparticles".

The encapsulated particles may be encapsulated in a silica glass as described in U.S. Patent No. 6,514,767. Other materials including but not limited to various types of polymers may also be used as an encapsulant consistent with the present invention. One crucial property of the particles in an effective LFI device is the ability to completely release from a dried state bond to the conjugate pad upon application of an unknown fluid sample. The use of silica encapsulated, polymer encapsulated or other encapsulated detection particles facilitates an easy release from the dry state. The flow characteristics of encapsulated particles may contribute to the development of a sharper read line in LFI devices designed for simple visual detection. As described in the example below, the enhanced flow characteristics of an encapsulated particle can increase the sensitivity of visually read LFI devices by at least seven times.

An alternative assay format featuring the use of encapsulated particles may be fabricated without the use of particles bound in a dry state to the conjugate pad. In particular, particles such as described herein may be incorporated into assay diluents. In this embodiment, described in detail in the examples below, analyte and detection particles can be mixed away from the test substrate and subsequently applied to the substrate. Accordingly the incubation time of the detection particles with the analyte can be controlled to achieve the most effective performance. In addition, the quantity of detection tags used in the assay can be precisely controlled. This may be of importance if there is limited space on a conjugation pad, or if multiple distinct detection tags are used in a multiplexed format. The alternative format of externally applied detection tags might eliminate the need for a conjugation pad, and thus address the problems associated with a pad such incomplete or non-uniform detection particle deposition or release.

In addition to the improved dispersal and flow characteristics achieved by preparing a LFI device with encapsulated particles, it is possible to tailor the surface properties of encapsulated particles to further enhance and control the flow properties of the particles. For example, particles can be coated with polymers that are recognized to have low non-specific binding characteristics such as dextrans or polyethylene glycol (PEG) derivatives to decrease particle interaction with exogenous proteins or the nitrocellulose substrate. The flow properties of the particles can also be altered by encapsulation with polymers selected to render the particles more hydrophilic or hydrophobic. Such changes are expected to cause significant changes in particle flow rates and can be used to tailor an assay to specific flow rate requirements.

Another possible method of affecting particle flow rate is to create coatings or encapsulations that significantly alter the particle size or hydrodynamic radius. For example, a larger particle, or one with a long-chain polymer encapsulation may have more interactions with a membrane and flow at a slower rate than a more compact particle. The nature of the coating will also influence the release rate of particles from the conjugate pad, both by defining the amount of interaction with the conjugate pad and the solubility of the particles in the assay diluents. For example, it could be desirable to slow down the release of particles from the conjugate pad by lowering particle solubility, thus allowing more time for the antigen to react at the test line prior to detection with antibody-coated particles. In addition, the particle surface charge could be a very important factor in the assay performance. By modifying or determining the isoelectric point of various particles through encapsulate selection; particle flow behavior in buffers of differing pHs could be impacted. This may allow the use of particles in a buffer that is not normally compatible with conjugates, but that has advantages for antibody-antigen binding or release of antigen from a complex within the sample matrix. Similarly, surfactants may be used to tailor the surface properties of particles thus affecting flow characteristics.

It is important to note that the tailoring of the particle's surface can be done in parallel with conjugation, for example, half the surface of a particle could be coated with PEG and half with an antibody. Alternatively surface tailoring can occur prior to conjugation of the antibody. Many of the desired effects can be achieved simultaneously, for example coating particles with an amine-terminated PEG to raise the isoelectric point, reduce non-specific binding and increase solubility.

Many substances may be suitable to encapsulate or coat particles to achieve some or all of the effects described above. Suitable substances include but are not limited to the following: silica glass, proteins, DNA, RNA, synthetic Polyaminoacids (Polylysine, Polyglutamic acid), Polyethylene glycols, block copolymer dendrimers, polyamides, polyethylenimines, polyacrylates and other natural polymers such as dextrans, other natural carbohydrate based polymers and surfactants. Other suitable coatings are discussed in United States Patent Application No. 11/622,915 entitled POLYMER COATED SERS NANOTAG, filed on January 12, 2007. The use of encapsulated detection particles may facilitate the use of oligonucleotides as binding moieties rather than the antibodies and antigens of a conventional LFI.

Prior art LFI devices featuring non-encapsulated detection particles require highly controlled substrate quality to ensure repeatable flow characteristics and accordingly repeatable and accurate test results. The rigorous substrate manufacturing standards which must be maintained add significantly to the cost of prior art devices. The use of encapsulated detection particles with enhanced flow characteristics as described above may relax the substrate quality standards necessary to achieve a select level of test performance. Furthermore the use of calibration and control techniques as described in detail below may offset the variability inherent in readily available substrates.

As discussed above, prior art LFI devices commonly utilize Au nanoparticles with a diameter ranging from 20-40 nm as a detection modality. Antibodies are adsorbed directly to the Au surface during the preparation of the LFI device. Since the steric hindrance of the Au nanoparticles limits the ability of passively adsorbed antibodies to effectively bind antigens present in the unknown sample, there is an upward limit associated with increased particle size. However, the use of larger particles would increase the sensitive of an LFI because the light scattering capabilities, and thus visibility, of the Au nanoparticles increases with size.

SERS nanotags in particular or generally any encapsulated particle can employ metal cores of any practical size without a loss in antibody activity. This is because the silica, polymer or other shell allows covalent coupling of antibodies which minimizes steric hindrance. An encapsulated particle with covalently coupled antibodies may also feature long tethers, branched polymer attachments or similar attachment structures such as hydrogels or PEG spacers which will make more surface are available to an antigen and allow more antibodies to be attached per particle. If antibodies are bound with a spacer any surface affects on antigen-antibody binding will be minimized. Furthermore, the shell provides an ideal substrate for selective modification to manipulate the nanoparticles' properties. Thus, the sensitive of a visually detected LFI can be increased by using larger encapsulated detection particles.

Encapsulation also allows the use of non-traditional shapes as detection particles. As used herein, a non-traditional shape includes any shape other than a sphere. Non-traditional shapes include, but are not limited to, prisms, cubes, pyramids, jacks, rectangular boxes, hollow shells or generally irregular shapes with flat or curved surfaces. Non-traditional shapes can be advantageous for use in a visually detected LFI device for the same reasons described above with respect to larger particles. Nontraditional shapes reflect light better than simple spheres and thus enhance the sensitivity of a visually detected LFI device. Prior art metal nanoparticles are unsuitable for use with an LFI device in nontraditional shapes because the dispersal and flow characteristics of non-encapsulated, non-spherical particles would severely compromise the functioning of the test. The enhanced mono-dispersal and flow characteristics of an encapsulated particle allow the use of nontraditional shapes.

Encapsulation also allows the use of metals other than Au as the detection modality of an LFI device. Au is favored in the prior art because of its favorable antibody adsorption characteristics. These characteristics are not necessarily shared by other metals, such as Ag, which would otherwise be more suitable for the detection particle of an LFI device because Ag is more reflective than Au. Because the silica, polymer or other encapsulant shell allows covalent coupling of antibodies to the encapsulated particle, the metal selected for the core becomes less critical. Accordingly, brighter, more reflective metals, such as Ag, can be used as the core of encapsulated collection particles.

The present invention may be implemented with encapsulated particles as defined in the enclosed claims. Certain advantages discussed below may be realized if the encapsulated particle produces a detectable spectrum of any type upon interrogation with light of a suitable wavelength. One type of encapsulated particle which is suitable for the implementation of the present invention is a SERS active nanoparticle such as the particles described in U.S. Patent No. 6,514,767. Other types of particles described herein include Raman active particles, Raman beads such as those described in Towards the DRED of Resin-Suported Combinatorial Libraries: A Non-Invasive Methodology Based on Bead Self-Encoding and Multispectral Imaging Fenniri, H.; Hedderich, H. G.; Haber, K. S.; Achkar, J; Taylor, B.; Ben-Amotz, D. Agnew. Chem. Int. Ed.; 2000; 39 (24); 4483-4485; and Barcoded Resins: A New Concept for Polymer-Supported Combinatorial Library Self-Deconvolution Fenniri, H.; Ding, L.; Ribbe, A. E.; Zyrianov, Y.J. Am. Chem. Soc.; (Communication); 2001; 123(33); 8151-8152. Other particles described herein include particulate substances having Raman activity, plus beads, particles or substances exhibiting any other type of detectable spectroscopic characteristics under known conditions.

As discussed in detail below, when SERS active nanoparticles were substituted for the simple Au nanoparticles of a prior art LFI device, substantially improved visual detection results were observed. In addition, the SERS spectrum of a reporter molecule associated with a SERS detection particle can be read by an appropriate detector, greatly enhancing the sensitivity, accuracy and flexibility of an LFI test. Furthermore, the preparation and analysis of highly multiplexed tests are possible when SERS or other spectroscopically active particles are used as the detection modality.

SERS allows detection of molecules attached to the surface of a *shingle* Au or Ag nanoparticle. A Raman enhancing metal that has associated or bound to it a Raman-active reporter molecule(s) is referred to as a SERS-active nanoparticle. Such SERS-active nanoparticles have utility as optical tags. However, SERS-active nanoparticles made from unencapsulated metals present formidable practical problems when used in such assays such as an LFI. Metal nanoparticles are exceedingly sensitive to aggregation in aqueous solution; once aggregated, it is not possible to re-disperse them. In addition, the chemical compositions of some Raman-active molecules are incompatible with the chemistries used to attach other molecules (such as proteins) to metal nanoparticles. This restricts the choices of Raman-active molecules, attachment chemistries, and other molecules to be attached to the metal nanoparticle.

SERS-active composite nanoparticles (SACNs) are comprised of a metal nanoparticle that has attached or associated with its surface one or more Raman-active molecules. This complex of Raman enhancing metal and analyte (referred to as a SERS-active metal nanoparticle) is then coated or encapsulated by an encapsulant. The encapsulant may be a glass material, and the SACN is referred to then as a glass-coated analyte loaded nanoparticle (GAN). SACNs may be provided by growing or otherwise placing a shell of a suitable encapsulant over a SERS-active metal nanoparticle core. The metal nanoparticle core is a Au or Ag sphere of any size, or another shape wherein multiple metal particles are included in the core.

The use of SERS active nanoparticles as the encapsulated detection modality of the present invention allows for intrinsic multiplexing. An LFI device consistent with the present invention may be prepared with a single capture line having a mixture of multiple capture antibodies. In addition, the conjugate pad may be prepared with several subsets of SERS nanotags conjugated to multiple separate detection antibodies. Each type of SERS detection particle would have its own type or SERS active reporter molecule. As described in detail below, multiple spectra may be obtained from the various SERS nanotags bound to a single capture line or point after the test is performed.

An LFI device featuring multiplexed SERS nanotags, another type of Raman active particle, Raman active molecules or any other type of particle which may produce a detectable spectrum upon interrogation as detection modality also has the ability to report calibrated result intensity. Calibrated test result intensity allows an LFI to function as more than a merely qualitative indicator. With calibrated intensity, quantitative measurements of the antigen of interest may be obtained. For example, in an embodiment where SERS nanotags are used as the detection modality, one or more of the various types of SERS nanotags in a multiplexed format may be interrogated as a positive control to verify that a necessary minimum particle flow quantity or rate has been achieved to assure meaningful results for the primary test particles.

Calibration can be accomplished by using a form of internal standard. One subset of the SERS nanotags (with SERS spectrum "A") is conjugated to a detection antibody against the antigen of interest ("unknown"). Another subset of SERS nanotags (with SERS spectrum "B") may be conjugated to a detection antibody against an antigen that is doped into the sample buffer at a known concentration. Capture antibodies for both antigens are mixed and deposited onto the lateral flow substrate, and both subsets of conjugated particles are deposited onto the conjugate pad. When a sample is run, the ratio of the two SERS spectra ("A"/"B") can be used to determine the concentration of the unknown. In a similar fashion, the "known" antigen can be omitted completely if the capture antibody directly binds the species conjugated to subset "B".

Calibration and the multiplexed analysis described above can be performed as automatic functions of a SERS analyzer specifically configured to read LFI results. A dedicated reader would include a receptacle configured to precisely receive and position an LFI card. The dedicated reader would also include a laser positioned to excite the SERS spectra of reporter molecules associated with SERS tags bound to a specific area on the LFI substrate. Thus, the traditional capture line of visually detected LFI device could be replaced with a capture point or capture dot which would allow the preparation of suitably accurate LFI devices without the use of excess collection modality or antibodies, resulting in lower overall unit cost. In other embodiments of the present invention, the ability to report a calibrated or quantified result can be achieved with encapsulated detection particles other than SERS nanotags. Suitable particles include but are not limited to Raman beads and other types of particles or molecules having detectable spectral characteristics upon interrogation with appropriate devices.

It will be readily apparent to those skilled in the art that a new type of LFI is possible when using detection modality having spectral characteristics which can be detected by a suitable device. For example, in an embodiment where SERS nanotags are used as the detection modality, LFI devices may be prepared which provide either two step or alternative step detection processes. In particular, a device may be detected visually or with a SERS spectrum reader or both, as described above. In use, an LFI device which provides a positive result upon visual inspection might be further read with the SERS spectrum reader to obtain preliminary quantitative data or to confirm the positive visual result. Particles other than SERS nanotags such as Raman beads may be used with similar results.

The use of SERS nanotags as detection modality also allows an LFI device to be prepared which receives whole blood for analysis. Prior art LFI devices are typically unsuitable for whole blood analysis because the intense coloration imparted by blood cells obscures any attempt to visually read the test by interfering with or blocking all light which might reflect from prior art Au nanoparticles. SERS nanotags, on the contrary, can be excited with light from the near infrared portion of the spectrum, at which wavelengths interference from hemoglobin would be minimized. In one embodiment of a whole blood suitable LFI device, a red transparent cover might be placed over the substrate to minimize the negative visual impact of the blood diffused through the test substrate. In another embodiment suitable for use with whole blood or other fluids, a filter with a pass band in the near IR wavelength range may be used allowing the passage of near IR light for interrogation of the SERS nanotags, yet simultaneously preventing potentially undesirable visual observation of the test substrate.

### EXAMPLES

The following examples are provided for illustrative purposes only and are not intended to limit the scope of the invention. Although the examples described below feature antigen and antibody binding events, comparable LFI systems could be prepared using oligonucleotides as binding moieties rather than the antibodies and antigens of a conventional LFI.

### Example 1.

Commercial lateral flow immunoassay (LFI) kits for respiratory syncytial virus (RSV) were obtained and modified to use SERS nanotags to detect the presence of RSV antigen. In addition, a number of kits were used as received in order to determine the sensitivity of the commercial product. To use SERS nanotags with the device, the conjugate pad within the kit was removed prior to use. In a typical experiment, a positive control was diluted with sample diluent from the kit (160 µl total volume) and mixed with SERS nanotags (15 µl of OD ∼5) that had been conjugated to an RSV detection antibody. After about 2 minutes, this mixture was added to the kit. The test was run at room temperature for about 15 min before visual reading and collection of the Raman spectra.

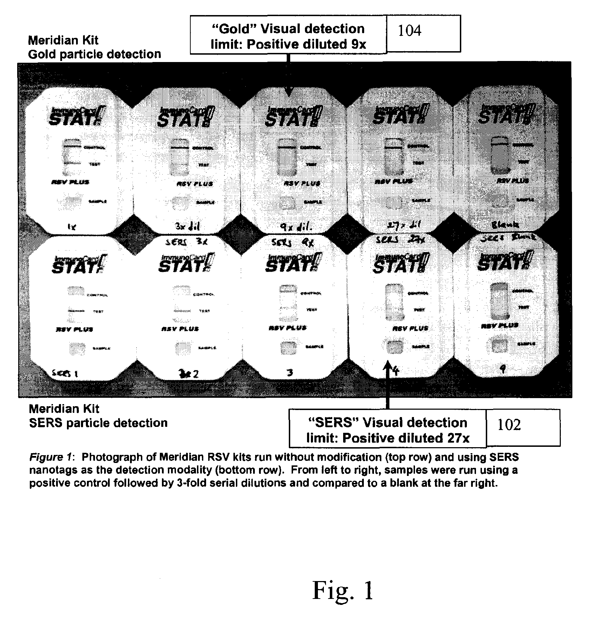

By visual detection alone, the sensitivity of the commercial tests versus the SERS nanotag-modified tests is shown in Fig. 1. The visual detection limit with SERS nanotags is as good or better than the commercial kit: a 27-fold diluted positive sample 102 resulted in a visible line whereas the pinkish-red line is fading with a 9-fold diluted sample 104 in the commercial format.

After visual detection, Raman spectra of the SERS-modified devices were collected. A commercially available laser and spectrometer was used. The laser power at the sample was approximately 200 mW, focused to a spot size of 100 µm. The acquisition was an average of four 1 sec integrations. Integration software was then used to analyze the contribution of the SERS nanotags to the overall signal (which also includes background from the nitrocellulose membrane).

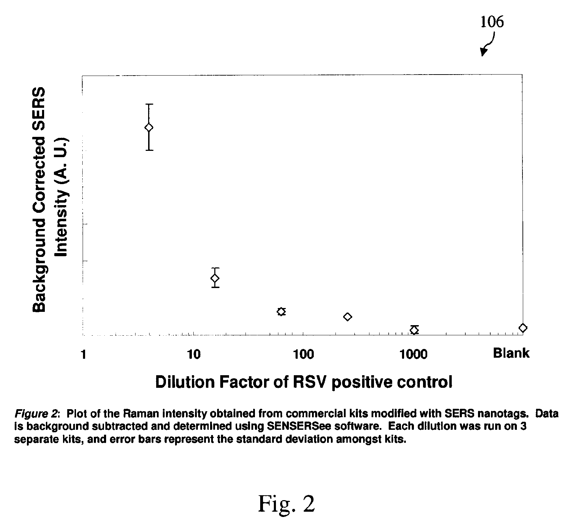

Fig. 2 shows a graph 106 of the relative Raman signal collected at the test line of a series of SERS modified kits incubated with serially diluted positive sample. A limit of detection (LOD) was determined corresponding to approximately a 400-fold diluted positive. Background measurements were taken on 8 locations from 8 different cards that were run with a blank (LOD was calculated as average background plus 3 standard deviations). The assay precision, determined on 3 replicate cards (read at test line) was better than 12%.

The data show minimal non-specific binding, and successful capture of the conjugate in the presence of antigen, with a SERS nanotag-based limit of detection that is increased by about 40 to 50 fold over the commercial product using visual detection of colloidal Au.

### Example 2. Singleplex Experiments:

As a preliminary step toward development of a multiplexed LFI test for RSV, Influenza A and Influenza B, a series of single-plex tests for each antigen were first developed. For each test, a pair of antibodies was purchased. Each antibody was conjugated to SERS nanotags of a selected "flavor" and tested on LFI strips as liquid conjugates, rather than dried conjugate pads, to determine the best combination of capture antibody on the nitrocellulose strip and detection antibody conjugated to the SERS nanotags. Once the best pair was found, the appropriate SERS nanotags were dried onto conjugate pads and assembled into complete tests.

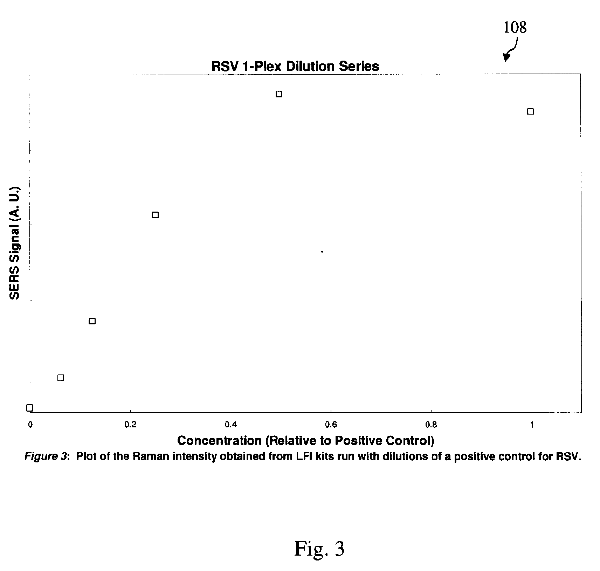

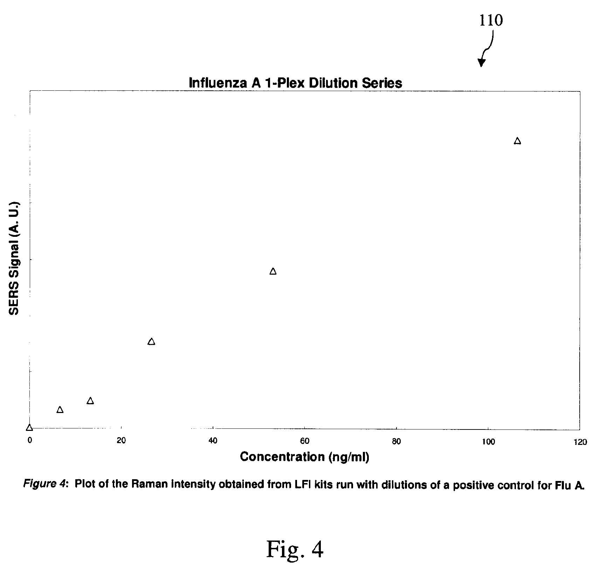

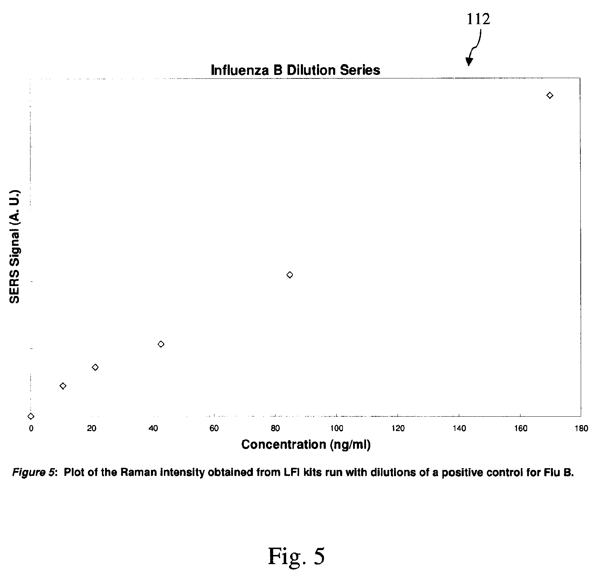

These tests were run using standards obtained for RSV, Influenza A and Influenza B. For RSV, the positive control (unknown concentration) was run without dilution and at various dilutions using a commercial LFI running buffer. The Influenza A and B antigens were of known concentration and were also diluted in the running buffer for testing. Cartridges were mounted on an XYZ-stage and the position of the sample was manually adjusted to obtain the best signal for each cartridge. Spectra were acquired using an 1 sec integration time a commercially available Raman spectrometer. Integration software was used to extract the amount of SERS signal from the spectra. Samples were analyzed only for the presence of nitrocellulose (background) or the single flavor of SERS nanotags used in each respective test. Results of these sensitivity tests are shown in graph 108 of Fig. 3 (RSV), graph 110 of Fig. 4 (Flu A) and graph 112 of Fig. 5 (Flu B).

Each of the three single-plex tests shows a fairly linear increase in signal with increasing antigen concentration, and importantly each has a very low signal with no antigen. Additionally, all tests were visually positive at the highest dilution of antigen and visually negative with the blank (running buffer only). By comparison, all of the commercial tests were negative at blank and the highest dilution of antigen. This indicates that the SERS nanotags are more sensitive with both a visual read and using a dedicated Raman reader. It is also worth emphasizing the experimental read time of just 1 sec, and no signal averaging. More sensitive results could be expected by averaging signals taken with a scanning laser across the test line (to minimize reader noise) and using longer integration times.

### Example 3. Multiplex Experiments:

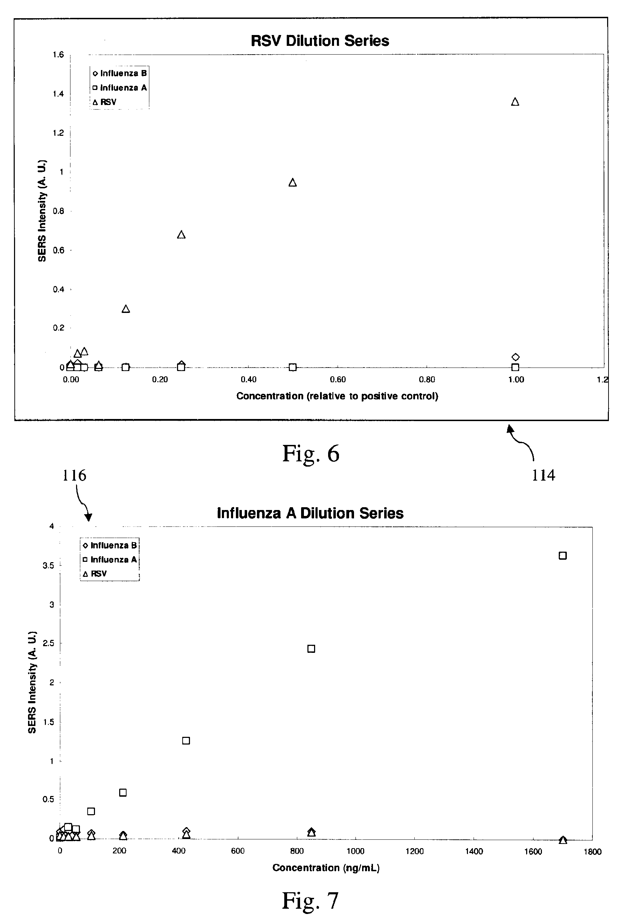

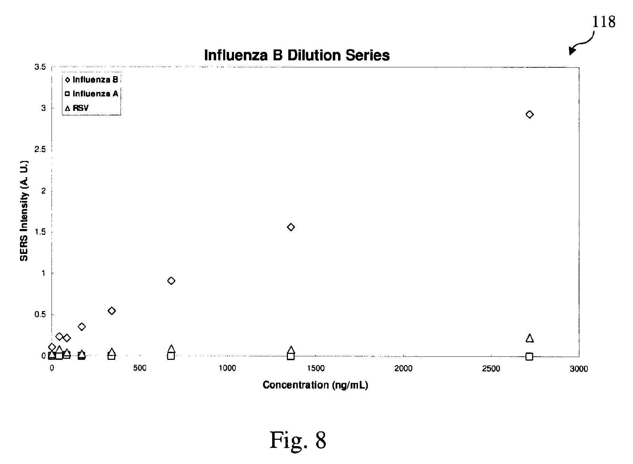

Once the appropriate conditions had been determined for the singleplex tests, the first attempt at a multiplexed LFI was essentially just a combination of all parts into one test. The 3-plex used the same d8-DPy-RSV conjugate as used for the singleplex test, but new conjugates had to be made for Flu A (DPy) and Flu B (BPE). All conjugates were mixed for deposition into conjugate pads. Likewise, all three capture antibodies were striped into a single line on the nitrocellulose cards. After assembly of the strips into cartridges, each antigen was tested using a similar concentration range. Only one antigen was used at a time for preliminary testing. However, data was analyzed against the presence of background nitrocellulose and all 3 flavors of tags. Also, the detection/interrogation instrument was modified with a dedicated cartridge holder. Thus, samples were inserted into the holder and spectra acquired without any manipulation to assure that the test line was being probed by the laser spot. For each antigen concentration, data for the amount of all 3 tags are plotted in graph 114 of Fig. 6 (RSV), graph 116 of Fig. 7 (Flu A) and graph 118 of Fig. 8 (Flu B). Background signals from all samples are very low, indicating that the flow is not significantly impeded by the capture of specifically bound tags. Note that the vertical scale is of equal magnitude for Figs. 6-8.

### Example 4. Multiplexed Assay Calibration Experiments:

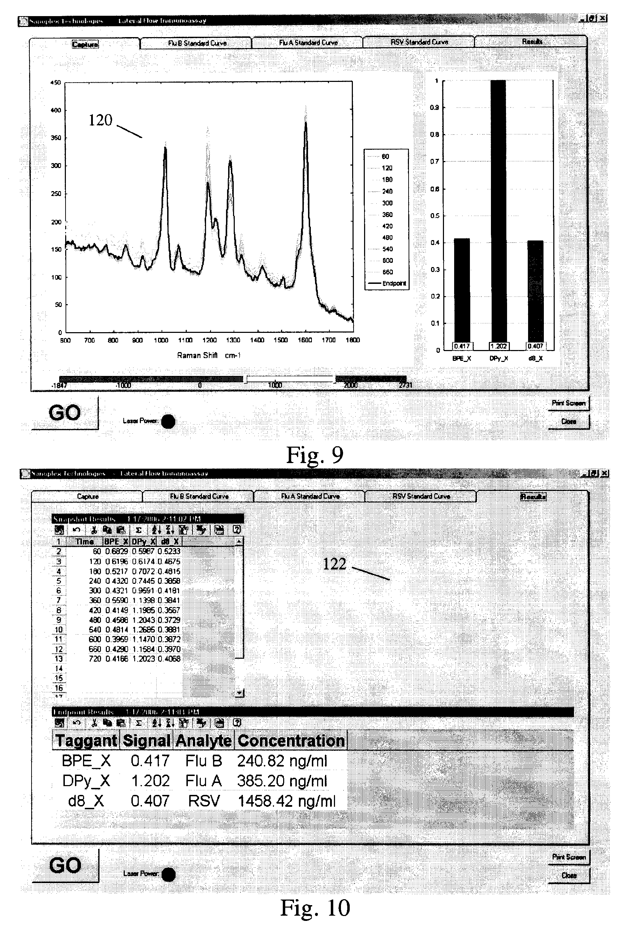

For each of the three analytes (Flu A, Flu B, and RSV), serial dilutions of a positive control were prepared and run on a multiplexed LFI kit to generate calibration curves. The calibration data and calibration curves are shown in Figs 9-13. All data points were acquired 15 minutes after the sample was added to a test kit. Standards were then used in conjunction with interrogation software to fit the data with the relative SERS signal from each conjugate type. Only the species of interest was plotted, and an appropriate curve was fit to the data using either linear or isotherm models.

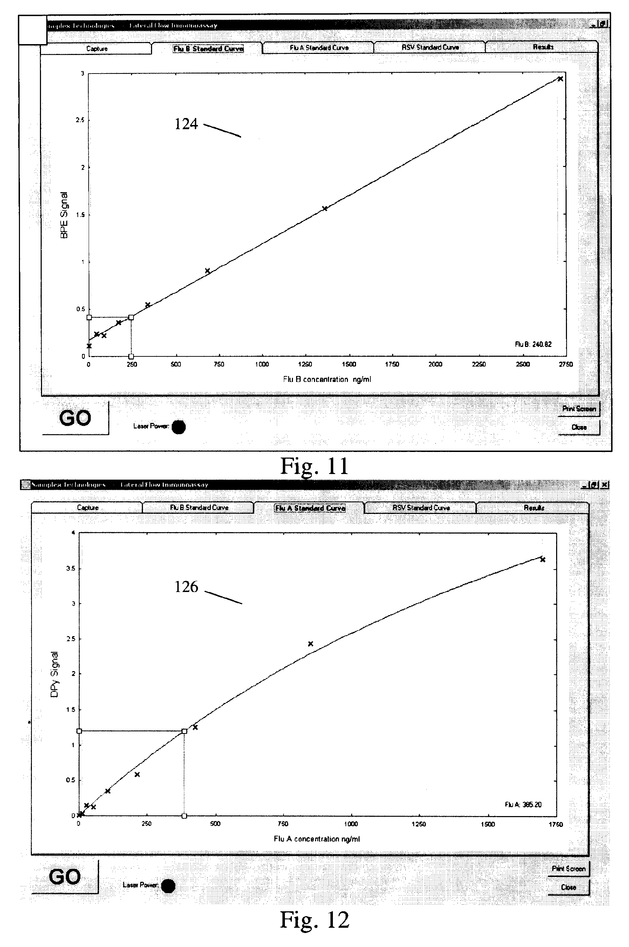

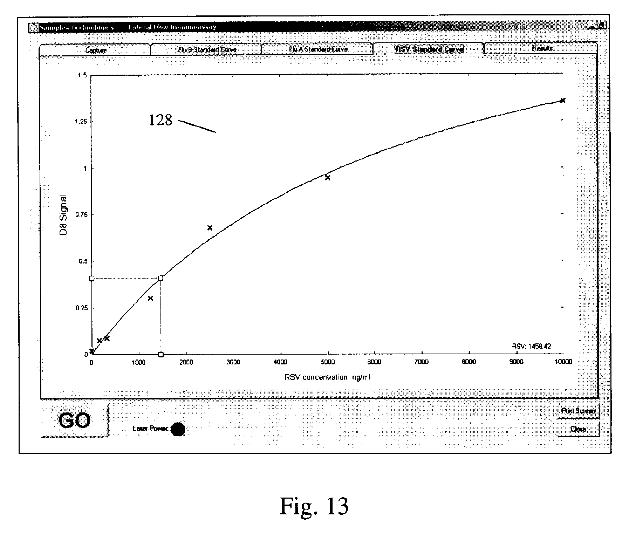

A sample was prepared with all three analytes present. This sample was run using interrogation software having a user interface optimized for use with an LFI assay format. The test cartridge holding the test substrates was inserted into the instrument 3 minutes after application of the sample. This allowed adequate time for the solution to begin diffusion into the nitrocellulose strip. Assuring that diffusion is occurring was important in the test format since the cartridge was inserted vertically for readings, which could cause liquid to spill across the substrate invalidating the test. The assay was then monitored every minute up to the 15 minute mark from addition of sample. Only the final data point was used for determination of analyte concentration. Screen capture 120 of Fig. 9 shows the acquired spectra at each time point, based upon an average of three 1 sec integrations, with the endpoint drawn in bold. Screen capture 122 of Fig. 10 illustrates the test data, which shows both relative levels at each time point as well as the final concentrations based on the calculated calibration curves. Screen capture 124 of Fig. 11 shows a calibration curve for Flu B and the position of the endpoint on the curve. Screen capture 126 of Fig 12 shows a calibration curve for Flu A and the position of the endpoint on the curve. Screen capture 128 of Fig 13 shows a calibration curve for RSV and the position of the endpoint on the curve.

### Example 5.

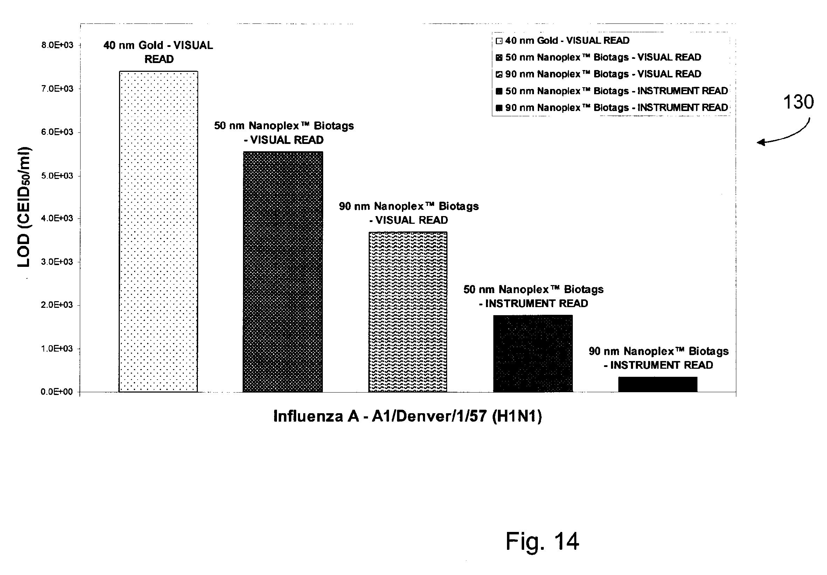

As described above, encapsulated detection particles have inherent sensitivity benefits for LFI applications over the traditionally used 40 nm gold conjugate. It is believed that much of this sensitivity comes from the ability to use larger gold (which is more easily seen) without losing antibody activity because antibodies can be covalently conjugated to the encapsulent shell. This concept was tested by developing 3 tests for Flu A that were identical other than the detection particle being used. The first sample used the standard 40 nm Au sol with passively adsorbed antibody. The other two samples used silica encapsulated SERS tags as described above, one sample having particles having 50 nm Au cores and the other with particles having 90 nm Au cores. An influenza A standard was purchased and used to test the sensitivity of each device. The devices were read by visual interrogation and those with SERS tags were also read using a scanning Raman reader. Results are shown in graph 130 of Fig. 14.

Tests prepared with 40 nm gold conjugates were reasonably sensitive, and comparable to results obtained from commercially available tests. However, 50 nm core SERS tags had a limit of detection that was about 1.3-fold more sensitive and 90 nm core particles were twice as sensitive. When the scanning Raman reader was used, 50 nm core particles were 4-fold more sensitive and 90 nm Au core particles were 21-fold more sensitive than usually read devices utilizing 40 nm conjugates.

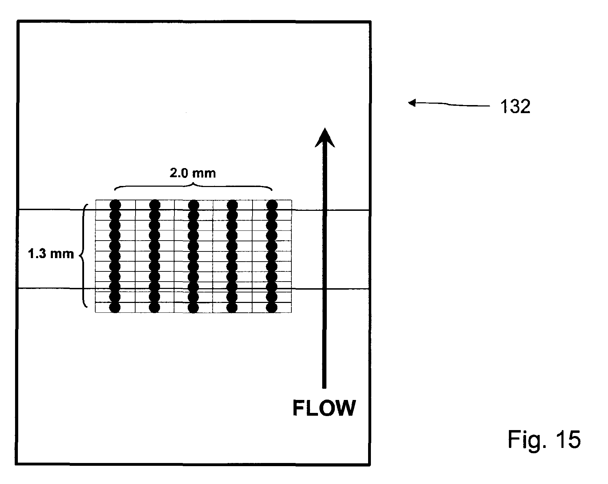

Though the core size of the particles were responsible for a portion of the deserved sensitivity boost, the use of a scanning Raman reader was also influential. Because of the inconsistencies that accompany lateral flow devices, each device has slightly different particle release and flow characteristics. A scanning Raman reader was found to help by allowing an area of the device to be mapped that contained both areas of the test line and adjacent areas that should not be contributing toward a positive result. Thus, devices were analyzed using a background subtraction technique. The most intense row (perpendicular to the flow of particles) was assumed to be the signal, and the least intense row was presumed to be the background. In this particular example, a scan was employed consisting of 11 rows (5 spots each) that were spaced at 130 µm intervals, as schematically shown by scan diagram 132 of Fig. 15. Other scan patterns could be utilized as well.

### Example 6.

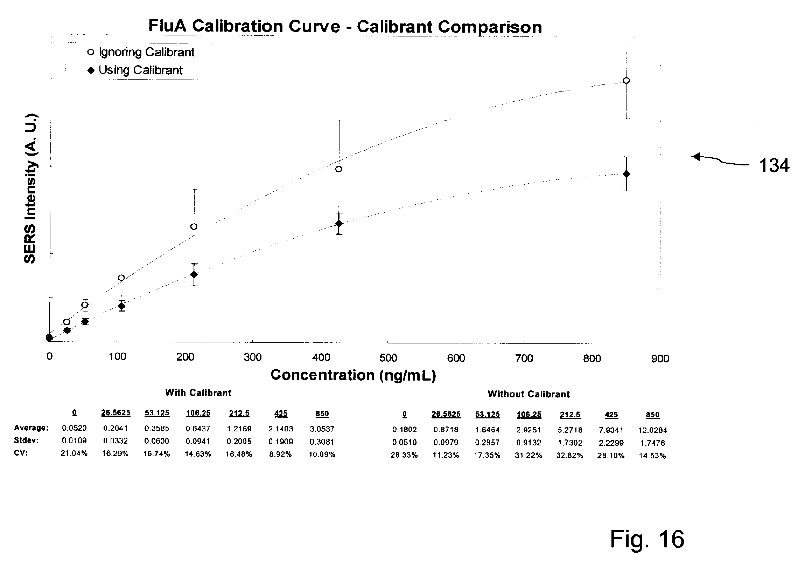

An encapsulated particle LFI assay with an internal calibration is demonstrated in Figure 16. Lateral flow devices were manufactured to contain 3 selected varieties of conjugates. Flu A and RSV monoclonal antibody conjugates were supplemented with SERS tag conjugates coated with normal rabbit IgG. A test line was striped onto a nitrocellulose membrane using a combination of Flu A and RSV capture antibodies and an anti-rabbit IgG antibody. Thus, even in the absence of any Flu A or RSV antigen, the rabbit IgG particles would be captured at the test line. In the presence of antigen, both rabbit IgG conjugates and Flu A and/or RSV conjugates will also be bound at the test line. In this manner, the calibration conjugant; (rabbit IgG conjugates) helps to account for variations in flow and conjugate release that are inherent to all lateral flow immunoassays. One possible test format would utilize a single read of the test line with a laser spot that is much smaller than the line. Thus, slight variations within a test line or introduced by manufacturing tolerances could lead to imprecision. The internal calibration can be used to compensate for imprecision inherent in any LFI test. Alternatively a laser scan of the entire line may be used with an averaged reading to increase precision.

Because of the suitability for multiplexed use of SERS tags, a spectrum can be readily analyzed to determine the amount of any number of different particles within a device. Figure 16 and the accompanying table demonstrate that if devices are analyzed without being normalized for the amount of calibrant, the CV for a given dilution can be up to 30%. However, if the same devices are normalized for calibrant, devices tend to have CVs of less than 20%.

Internal calibration can be extended to function as a control for the test. Most LFIs use two lines, one of which is intended to always develop a signal indicating proper flow of particles. Because an instrument is decoupling signal due to presence of antigen from the signal from a calibrant, merely detecting a calibrant can function to ensure the test was run and read properly.

While the invention has been particularly shown and described with reference to a number of embodiments, it would be understood by those skilled in the art that changes in the form and details may be made to the various embodiments disclosed herein and that the various embodiments disclosed herein are not intended to act as limitations on the scope of the claims.

## Claims

1. A method of performing a lateral flow immunoassay (LFI) comprising:
providing a substrate;
providing encapsulated detection particles conjugated to a detection antibody;
providing capture antibodies on a test portion of the substrate wherein both the detection antibodies and the capture antibodies have binding affinity for an antigen of interest;
binding the encapsulated particles to the antigen of interest with the detection antibody;
diffusing a fluid sample and encapsulated particles bound to the antigen through the substrate to the test portion of the substrate;
capturing the encapsulated particles and antigens at the test portion of the substrate with the capture antibodies; and
observing the encapsulated particles at the test portion of the substrate;
wherein
the encapsulated detection particles have a SERS-active metal nanoparticle core selected from Au and/or Ag;
multiple metal particles are included in the core; and
the metal nanoparticles have attached or associated with their surface one or more Raman-active molecules.

2. The method of claim 1 wherein the binding step occurs on the conjugation pad; or away from the substrate, prior to diffusing the fluid sample and encapsulated particles through the substrate; or while diffusing the fluid sample and encapsulated particles through the substrate.

3. The method of claim 1 or 2 wherein the encapsulant comprises a material selected from a group consisting of silica, glass, proteins, DNA, RNA, synthetic polyaminoacids, polylysine, polyglutamic acid, polyethylene glycols, block copolymer dendrimers, polyamides, polyethylenimines, polyacrylates, other natural polymers, dextrans, other natural carbohydrate based polymers and surfactants.

4. The method of any one of claims 1 to 3, wherein the encapsulated detection particles have a Ag core.

5. The method of any one of claims 1 to 4, wherein the encapsulated detection particles have a shape that is not substantially spherical, preferably a shape selected from a prism, a pyramid, a cube, a box, a jack, a hollow shell and an irregular shape.

6. The method of any one of claims 1 to 5, wherein the encapsulated detection particles have a detectable spectrum upon illumination with light of a select wavelength.

7. The method of any one of claims 1 to 6 wherein the observing step comprises:
optionally visually observing the test portion of the substrate;
illuminating the SACNs bound to the test portion with light capable of exciting the Raman reporter molecule, preferably the light used in the illumination step has a near infrared wavelength; and
detecting the Raman spectrum of the Raman reporter molecule.

8. The method of any one of claims 1-7 wherein the sample comprises whole blood.

9. The method of any one of claims 1-8, further comprising:
providing more than one type of SACN where each type of SACN has a separate type of Raman active reporter molecule and each type of SACN is conjugated to a distinct type of detection antibody; and
providing more than one type of capture antibody on a test portion of the substrate.

10. The method of claim 9, wherein the observing step further comprises:
illuminating the SACNs bound to the test portion with light capable of exciting each type of Raman reporter molecule; and
detecting the Raman spectrum of each type of Raman reporter molecule.

11. The method of claim 10, further comprising calibrating the Raman spectrum of one type of reporter molecule with the Raman spectrum of another type of reporter molecule.

12. A lateral flow immunoassay (LFI) device comprising:
a substrate having a conjugation portion and a test portion;
encapsulated detection particles conjugated to a detection antibody as defined in any one of claims 1, and 3 to 6 operatively associated with the conjugation portion;
capture antibodies operatively associated with the test portion; and
one of a red filter and near infrared filter associated with the substrate.

13. The LFI device of claim 12, wherein the LFI device further comprises more than one type of SACN, where each type of SACN has a separate type of Raman active reporter molecule and each type of SACN is conjugated to a distinct type of detection antibody, and optionally the LFI device further comprises a Raman Spectrum reader.

14. A method of performing a lateral flow immunoassay (LFI) comprising:
providing a substrate;
providing encapsulated detection particles conjugated to a detection oligonucleotide;
providing capture oligonucleotides on a test portion of the substrate wherein both the detection oligonucleotides and the capture oligonucleotides are complimentary to a target oligonucleotide of interest;
binding the encapsulated particles to the target oligonucleotide of interest with the detection oligonucleotide;
diffusing a fluid sample and encapsulated particles bound to the oligonucleotide of interest through the substrate to the test portion of the substrate;
capturing the encapsulated particles and oligonucleotide of interest at the test portion of the substrate with the capture oligonucleotides; and
observing the encapsulated particles at the test portion of the substrate
wherein
the encapsulated detection particles have a SERS-active metal nanoparticle core selected from Au and/or Ag;
multiple metal particles are included in the core; and
the metal nanoparticles have attached or associated with their surface one or more Raman-active molecules.

15. The method of any one of claims 1 to 11, or claim 14, wherein the encapsulated detection particles comprise an encapsulant and a coating.

16. The method of claim 15, wherein the coating comprises a material selected from a group consisting of silica, glass, proteins, DNA, RNA, synthetic Polyaminoacids, Polylysine, Polyglutamic acid, Polyethylene glycols, block copolymer dendrimers, polyamides, polyethylenimines, polyacrylates, other natural polymers, dextrans, other natural carbohydrate based polymers and surfactants.

## Patentansprüche

1. Verfahren zum Durchführen eines Querfluss-Immuntests (QFI), umfassend:
Bereitstellen eines Substrats;
Bereitstellen eingekapselter, mit einem Erkennungs-Antikörper verbundenen Erkennungsteilchen;
Bereitstellen von Fänger-Antikörpern auf einem Prüfabschnitt des Substrats, wobei sowohl die Erkennungs-Antikörper als auch die Fänger-Antikörper Bindungsaffinität für ein Antigen von Interesse aufweisen;
Binden der eingekapselten Teilchen mit dem Erkennungs-Antikörper an das Antigen von Interesse;
Diffundieren einer Fluidprobe und an das Antigen gebundene eingekapselte Teilchen durch das Substrat zum Prüfabschnitt des Substrats;
Aufnehmen der eingekapselten Teilchen und Antigene mit den Fänger-Antikörpern am Prüfabschnitt des Substrats; und
Beobachten der eingekapselten Teilchen am Prüfabschnitt des Substrats;
wobei
die eingekapselten Erkennungsteilchen einen SERS-aktiven Metallnanopartikelkern aufweisen, ausgewählt aus Au und/oder Ag;
mehrere Metallteilchen im Kern enthalten sind; und
die Metallnanopartikel ein oder mehrere Raman-aktive Moleküle an ihrer Oberfläche befestigt oder damit assoziiert aufweisen.

2. Verfahren nach Anspruch 1, wobei der Bindungsschritt auf dem Verbindungsfeld stattfindet; oder vom Substrat entfernt, vor dem Diffundieren der Fluidprobe und eingekapselten Teilchen durch das Substrat; oder während des Diffundierens der Fluidprobe und eingekapselten Teilchen durch das Substrat.

3. Verfahren nach Anspruch 1 oder 2, wobei das Einkapselungsmittel ein Material umfasst, ausgewählt aus einer Gruppe bestehend aus Siliciumdioxid, Glas, Proteinen, DNS, RNS, synthetischen Polyaminosäuren, Polylysin, Polyglutaminsäure, Polyethylenglykolen, Block-Copolymerdendrimeren, Polyamiden, Polyethyleniminen, Polyacrylaten, weiteren natürlichen Polymeren, Dextranen, weiteren natürlichen auf Kohlenhydrat basierenden Polymeren und Tensiden.

4. Verfahren nach einem der Ansprüche 1 bis 3, wobei die eingekapselten Erkennungsteilchen einen Ag-Kern aufweisen.

5. Verfahren nach einem der Ansprüche 1 bis 4, wobei die eingekapselten Erkennungsteilchen eine Form aufweisen, die im Wesentlichen nicht kugelförmig ist, vorzugsweise eine Form, ausgewählt aus einem Prisma, einer Pyramide, einem Würfel, einem Kasten, einem Stab, einer hohlen Schale und einer unregelmäßigen Form.

6. Verfahren nach einem der Ansprüche 1 bis 5, wobei die eingekapselten Erkennungsteilchen bei Beleuchtung mit Licht einer ausgewählten Wellenlänge ein detektierbares Spektrum aufweisen.

7. Verfahren nach einem der Ansprüche 1 bis 6, wobei der Beobachtungsschritt umfasst:
wahlweise visuelles Beobachten des Prüfabschnitts des Substrats;
Beleuchten der an den Prüfabschnitt gebundenen SACNs mit Licht, in der Lage dazu, das Raman-Reportermolekül anzuregen, vorzugsweise wobei das im Beleuchtungsschritt verwendete Licht eine Wellenlänge im nahen Infrarot aufweist; und
Detektieren des Ramanspektrums des Raman-Reportermoleküls.

8. Verfahren nach einem der Ansprüche 1-7, wobei die Probe Vollblut umfasst.

9. Verfahren nach einem der Ansprüche 1-8, ferner umfassend:
Bereitstellen von mehr als einer Sorte von SACN, wobei jede Sorte von SACN eine separate Sorte Raman-aktives Reportermolekül aufweist und jede Sorte von SACN mit einer individuellen Sorte Erkennungs-Antikörper verbunden ist; und
Bereitstellen von mehr als einer Sorte Fänger-Antikörper auf einem Prüfabschnitt des Substrats.

10. Verfahren nach Anspruch 9, wobei der Beobachtungsschritt ferner umfasst:
Beleuchten der an den Prüfabschnitt gebundenen SACNs mit Licht, in der Lage dazu, jede Sorte Raman-Reportermolekül anzuregen; und
Detektieren des Ramanspektrums von jeder Sorte Raman-Reportermolekül.

11. Verfahren nach Anspruch 10, ferner umfassend Kalibrieren des Ramanspektrums von einer Sorte Reportermolekül mit dem Ramanspektrum einer anderen Sorte Reportermolekül.

12. Querfluss-Immuntest (QFI)-Vorrichtung, umfassend:
ein Substrat mit einem Verbindungsabschnitt und einem Prüfabschnitt;
eingekapselte Erkennungsteilchen verbunden mit einem Erkennungs-Antikörper wie in einem der Ansprüche 1 und 3 bis 6 definiert, wirkverbunden mit dem Verbindungsabschnitt assoziiert;
mit dem Prüfabschnitt wirkverbunden assoziierte Fänger-Antikörper; und
eines von einem mit dem Substrat assoziierten Rotfilter und Nahinfrarotfilter.

13. QFI-Vorrichtung nach Anspruch 12, wobei die QFI-Vorrichtung ferner mehr als eine Sorte SACN umfasst, wobei jede Sorte SACN ein separates Raman-aktives Reportermolekül aufweist und jede Sorte SACN mit einer individuellen Sorte Erkennungs-Antikörper verbunden ist und die QFI-Vorrichtung ferner wahlweise ein Ramanspektrum-Lesegerät umfasst.

14. Verfahren zum Durchführen eines Querfluss-Immuntests (QFI), umfassend:
Bereitstellen eines Substrats;
Bereitstellen eingekapselter, mit einem Erkennungs-Oligonukleotid verbundenen Erkennungsteilchen;
Bereitstellen von Fänger-Oligonukleotiden auf einem Prüfabschnitt des Substrats, wobei sowohl die Erkennungs-Oligonukleotide als auch die Fänger-Oligonukleotide zu einem ZielOligonukleotid von Interesse komplementär sind;
Binden des eingekapselten Teilchens an das Ziel-Oligonukleotid von Interesse mit dem Erkennungs-Oligonukleotid;
Diffundieren einer Fluidprobe und an das Oligonukleotid gebundene eingekapselte Teilchen durch das Substrat zum Prüfabschnitt des Substrats;
Aufnehmen der eingekapselten Teilchen und des Oligonukleotids von Interesse am Prüfabschnitt des Substrats mit den Fänger-Oligonukleotiden; und
Beobachten der eingekapselten Teilchen am Prüfabschnitt des Substrats
wobei
die eingekapselten Erkennungsteilchen einen SERS-aktiven Metallnanopartikelkern aufweisen, ausgewählt aus Au und/oder Ag;
mehrere Metallteilchen im Kern enthalten sind; und
die Metallnanopartikel ein oder mehrere Raman-aktive Moleküle an ihrer Oberfläche befestigt oder damit assoziiert aufweisen.

15. Verfahren nach einem der Ansprüche 1 bis 11 oder Anspruch 14, wobei die eingekapselten Erkennungsteilchen ein Einkapselungsmittel und eine Beschichtung umfassen.

16. Verfahren nach Anspruch 15, wobei die Beschichtung ein Material umfasst, ausgewählt aus einer Gruppe, bestehend aus Siliciumdioxid, Glas, Proteinen, DNS, RNS, synthetischen Polyaminosäuren, Polylysin, Polyglutaminsäure, Polyethylenglykolen, Block-Copolymerdendrimeren, Polyamiden, Polyethyleniminen, Polyacrylaten, weiteren natürlichen Polymeren, Dextranen, weiteren natürlichen auf Kohlenhydraten basierenden Polymeren und Tensiden.

## Revendications

1. Procédé d'exécution d'un immunodosage à écoulement latéral (LFI) comprenant :
la fourniture d'un substrat ;
la fourniture de particules de détection encapsulées conjuguées à un anticorps de détection ;
la fourniture d'anticorps de capture sur une partie de test du substrat, dans lequel tant les anticorps de détection que les anticorps de capture possèdent une affinité de liaison pour un antigène d'intérét ;
la liaison des particules encapsulées à l'antigène d'intérêt avec l'anticorps de détection ;
la diffusion d'un échantillon de fluide et des particules encapsulées liées à l'antigène à travers le substrat vers la partie de test du substrat ;
la capture des particules et antigènes encapsulés au niveau de la partie de test du substrat avec les anticorps de capture ; et
l'observation des particules encapsulées au niveau de la partie de test du substrat ;
dans lequel
les particules de détection encapsulées possèdent un noyau nanoparticulaire métallique actif en spectrométrie laser de l'effet Raman exalté de surface (SERS) choisi parmi Au et/ou Ag ;
plusieurs particules métalliques sont incluses dans le noyau ; et
les nanoparticules métalliques possèdent fixées ou associées à leur surface une ou plusieurs molécules à effet Raman.

2. Procédé selon la revendication 1, dans lequel l'étape de liaison se produit sur le tampon de conjugaison ; ou à l'écart du substrat, avant la diffusion de l'échantillon de fluide et des particules encapsulées à travers le substrat ; ou pendant la diffusion de l'échantillon de fluide et des particules encapsulées à travers le substrat.

3. Procédé selon la revendication 1 ou 2, dans lequel l'agent d'encapsulation comprend un matériau choisi dans un groupe constitué de silice, verre, protéines, ADN, ARN, acides polyaminés synthétiques, polylysine, acide polyglutamique, polyéthylène glycols, dendrimères de copolymère séquencé, polyamides, polyéthylène-imines, polyacrylates, autres polymères naturels, dextranes, autres polymères naturels à base de glucide et agents tensioactifs.

4. Procédé selon l'une quelconque des revendications 1 à 3, dans lequel les particules de détection encapsulées possèdent un noyau en Ag.

5. Procédé selon l'une quelconque des revendications 1 à 4, dans lequel les particules de détection encapsulées ont une forme qui n'est pas sensiblement sphérique, de préférence une forme choisie parmi un prisme, une pyramide, un cube, une boîte, un plot, une enveloppe creuse et une forme irrégulière.

6. Procédé selon l'une quelconque des revendications 1 à 5, dans lequel les particules de détection encapsulées possèdent un spectre détectable lors d'un éclairage avec une lumière d'une longueur d'onde sélectionnée.

7. Procédé selon l'une quelconque des revendications 1 à 6, dans lequel l'étape d'observation comprend :
l'observation visuelle éventuelle de la partie de test du substrat ;
l'éclairage des nanoparticules composites actives en SERS (SACN) liées à la partie de test avec une lumière capable d'exciter la molécule rapporteuse de Raman, de préférence la lumière utilisée dans l'étape d'éclairage possédant une longueur d'onde dans l'infrarouge proche ; et
la détection du spectre Raman de la molécule rapporteuse de Raman.

8. Procédé selon l'une quelconque des revendications 1 à 7, dans lequel l'échantillon comprend du sang total.

9. Procédé selon l'une quelconque des revendications 1 à 8, comprenant en outre :
la fourniture de plus d'un type de SACN, où chaque type de SACN possède un type indépendant de molécule rapporteuse active en Raman et chaque type de SACN est conjugué à un type distinct d'anticorps de détection ; et
la fourniture de plus d'un type d'anticorps de capture sur une partie de test du substrat.

10. Procédé selon la revendication 9, dans lequel l'étape d'observation comprend en outre :
l'éclairage des SACN liés à la partie de test avec une lumière capable d'exciter chaque type de molécule rapporteuse de Raman ; et
la détection du spectre Raman de chaque type de molécule rapporteuse de Raman.

11. Procédé selon la revendication 10, comprenant en outre l'étalonnage du spectre Raman d'un type de molécule rapporteuse avec le spectre Raman d'un autre type de molécule rapporteuse.

12. Dispositif d'immunodosage à écoulement latéral (LFI), comprenant :
un substrat possédant une partie de conjugaison et une partie de test ;
des particules de détection encapsulées conjuguées à un anticorps de détection tel que défini dans l'une quelconque des revendications 1 et 3 à 6, associées fonctionnellement à la partie de conjugaison ;
des anticorps de capture associés fonctionnellement à la partie de test ; et
un filtre parmi un filtre rouge et un filtre infrarouge proche, associé au substrat.

13. Dispositif de LFI selon la revendication 12, où le dispositif de LFI comprend en outre plus d'un type de SACN, où chaque type de SACN possède un type indépendant de molécule rapporteuse active en Raman et chaque type de SACN est conjugué à un type distinct d'anticorps de détection et éventuellement le dispositif de LFI comprend en outre un lecteur de spectre Raman.

14. Procédé d'exécution d'un immunodosage à écoulement latéral (LFI) comprenant :
la fourniture d'un substrat ;
la fourniture de particules de détection encapsulées conjuguées à un oligonucléotide de détection ;
la fourniture d'oligonucléotides de capture sur une partie de test du substrat, dans lequel tant les oligonucléotides de détection que les oligonucléotides de capture sont complémentaires à un oligonucléotide cible d'intérét ;
la liaison des particules encapsulées à l'oligonucléotide cible d'intérêt avec l'oligonucléotide de détection ;
la diffusion d'un échantillon de fluide et des particules encapsulées liées à l'oligonucléotide d'intérêt à travers le substrat vers la partie de test du substrat ;
la capture des particules et de l'oligonucléotide d'intérêt, encapsulés au niveau de la partie de test du substrat avec les oligonucléotides de capture ; et
l'observation des particules encapsulées au niveau de la partie de test du substrat
dans lequel
les particules de détection encapsulées possèdent un noyau nanoparticulaire métallique actif en spectrométrie laser de l'effet Raman exalté de surface (SERS) choisi parmi Au et/ou Ag ;
plusieurs particules métalliques sont incluses dans le noyau ; et
les nanoparticules métalliques possèdent fixées ou associées à leur surface une ou plusieurs molécules à effet Raman.

15. Procédé selon l'une quelconque des revendications 1 à 11 ou la revendication 14, dans lequel les particules de détection encapsulées comprennent un agent d'encapsulation et un revêtement.

16. Procédé selon la revendication 15, dans lequel le revêtement comprend un matériau choisi dans un groupe constitué de silice, verre, protéines, ADN, ARN, acides polyaminés synthétiques, polylysine, acide polyglutamique, polyéthylène glycols, dendrimères de copolymère séquencé, polyamides, polyéthylène-imines, polyacrylates, autres polymères naturels, dextranes, autres polymères naturels à base de glucide et agents tensioactifs.
